(19) **Europäisches Patentamt / European Patent Office / Office européen des brevets**

(11) **EP 3 777 516 A1**

(12) # EUROPEAN PATENT APPLICATION
published in accordance with Art. 153(4) EPC

(43) Date of publication:
**17.02.2021 Bulletin 2021/07**

(21) Application number: **19778024.0**

(22) Date of filing: **22.03.2019**

(51) Int Cl.:
*A01G 7/00* (2006.01)      *G01N 21/31* (2006.01)

(86) International application number:
**PCT/JP2019/012260**

(87) International publication number:
**WO 2019/188848 (03.10.2019 Gazette 2019/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.03.2018 JP 2018062412**

(71) Applicants:
• **National University Corporation Ehime University**
  **Ehime 790-8577 (JP)**
• **Plant Data Co., Ltd.**
  **Matsuyama-shi, Ehime 790-8566 (JP)**

(72) Inventors:
• **TAKAYAMA, Kotaro**
  **Matsuyama-shi, Ehime 790-8566 (JP)**
• **SHIMOMOTO, Kota**
  **Matsuyama-shi, Ehime 790-8566 (JP)**
• **NISHINA, Hiroshige**
  **Matsuyama-shi, Ehime 790-8566 (JP)**
• **TAKAHASHI, Noriko**
  **Matsuyama-shi, Ehime 790-8566 (JP)**
• **INABA, Kazue**
  **Matsuyama-shi, Ehime 790-8566 (JP)**

(74) Representative: **Gulde & Partner**
**Patent- und Rechtsanwaltskanzlei mbB**
**Wallstraße 58/59**
**10179 Berlin (DE)**

(54) **PHOTOSYNTHESIS RATE MEASUREMENT SYSTEM**

(57) The present invention provides a photosynthesis rate measurement system capable of measuring carbon dioxide concentration in order to calculate the photosynthesis rate of plants with a simple configuration. The present invention is a photosynthesis rate measurement system, including a covering portion covering target plants, a sensor housing, and a sensor alternately measuring carbon dioxide concentrations inside and outside the covering portion. The sensor is provided in the sensor housing, and an air stirring portion to improve the sensor's response is equipped in the sensor housing.

Fig. 1

EP 3 777 516 A1

## Description

## Technical Field

The present invention relates to a photosynthesis rate measurement system capable of measuring a photosynthesis rate of plants.

## Background Art

A system for measuring a photosynthesis rate of a plant is known.

Patent Literature 1 discloses an evaluation system capable of evaluating a photosynthetic sample accurately and more easily.

## Citation List

## Patent Literature

Patent Literature 1: JP-A-2017-44531

## Summary of Invention

## Technical Problem

The present invention provides a photosynthesis rate measurement system capable of measuring a carbon dioxide concentration in order to calculate a photosynthesis rate of a plant with a simple configuration.

## Solution to Problem

Hereinafter, various embodiments of the present invention will be exemplified. The embodiments described below can be combined with each other.

The present invention provides a photosynthesis rate measurement system of a plant, comprising: a covering portion covering a target plant, a sensor housing, and a sensor in the sensor housing alternately measuring carbon dioxide concentrations inside and outside the covering portion, wherein an air stirring portion to enhance the mixing of the air in the sensor housing is equipped inside the sensor housing.

According to the present invention, a photosynthesis rate measurement system comprising the covering portion covering the target plant, the sensor housing, and the sensor alternately measuring carbon dioxide concentrations inside and outside the covering portion is provided. Specifically, the sensor is provided in the sensor housing. Further, the air stirring portion stirring the air exposing the sensor is provided inside the sensor housing. The air stirring portion is provided, so that the air flow is stirred, and the contact between the sensor and the air is promoted. Consequently, the response speed of the sensor is improved.

Hereinafter, various embodiments of the present invention will be exemplified. The embodiments described below can be combined with each other.

Preferably, the sensor measures the difference in the carbon dioxide concentrations between inflow air and outflow air of the covering portion, and the air stirring portion is provided to improve response speed of the sensor due to changes in the carbon dioxide concentration in the air on an inflow side or an outflow side of the air into the covering portion.

Preferably, the air stirring portion is provided to face a surface where the sensor and the air are in contact with each other inside the sensor housing.

Preferably, the covering portion comprises a canopy and a covering sheet, the canopy is arranged above the target plant, the covering sheet is arranged to cover a side of the target plant, and the covering sheet is configured to comprise an overlapped part covered by a plurality of sheets.

Preferably, the system further comprises a lid and a string, wherein the canopy is provided with a hole, the lid comprises an insertion portion through which the string can be inserted and a slit extending from an outer edge of the lid to the insertion portion, the lid is arranged such that the insertion portion overlaps the hole and arranged to cover the hole in a plan view, and the string is inserted through the hole via the insertion portion.

Preferably, the slit is configured such that slit's width increases toward the edge of the lid. Preferably, a diameter r of the hole and a diameter R of the lid satisfy a relationship of $2 < R/r$. Preferably, the canopy is provided with a ventilation fan.

## Brief Description of Drawings

Fig. 1 is a perspective view showing a system 100 according to an embodiment of the present invention.
Fig. 2 is a perspective view showing an overlapped part of a covering portion 1.
Fig. 3 is a plan view showing an example of a canopy 10.
Fig. 4A is a plan view showing a positional relationship between a lid 2 and a hole 11.
Fig. 4B is a plan view showing the shape of the lid 2 in detail.
Fig. 5 is a schematic view showing the arrangement of a sensor 4.
Fig. 6A is a schematic view showing, from a direction X in Fig. 1, a state where the sensor 4 is arranged in the system 100.
Fig. 6B is a schematic view showing a state where air M is sucked in through a route different from a route in Fig. 6A.
Fig. 7A is a schematic view showing air flow in a sensor housing 5 when an air stirring portion 51 is not provided.
Fig. 7B is a schematic view showing the air flow in the sensor housing 5 when the air stirring portion 51 is provided.

Fig. 8 is an example of experimental results for explaining the necessity of the air stirring portion 51.

Fig. 9 is an example of experimental results for explaining the necessity of the air stirring portion 51.

**Description of Embodiments**

Hereinafter, embodiments of the present invention will be described with reference to the drawings. Various characteristics in the embodiments described below can be combined with each other.

1. System 100

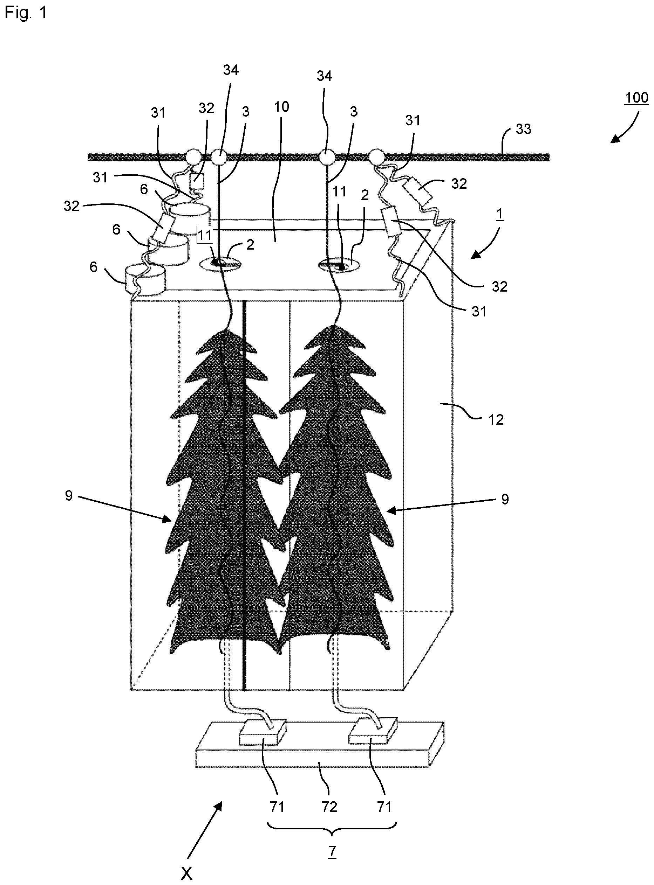

A photosynthesis rate measurement system of a plant according to an embodiment of the present invention is described with reference to Fig. 1 to Fig. 7B. Hereinafter, the photosynthesis rate measurement system is referred to as a system 100.

The system 100 is configured to measure the carbon dioxide concentration around a plant 9 and to calculate a photosynthesis rate of the plant 9 from the carbon dioxide concentration. The photosynthesis rate of the plant 9 can be calculated in real time by means of the system 100 according to the present embodiment. The calculated result can be applied to environmental control for increasing the photosynthesis rate, for example, by adjusting the carbon dioxide concentration around the plant 9.

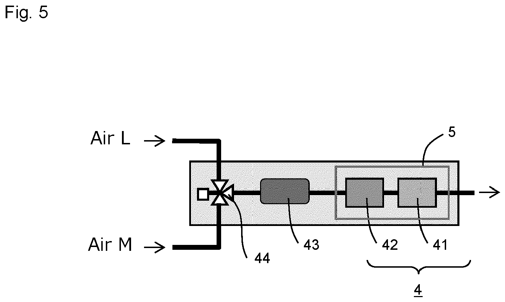

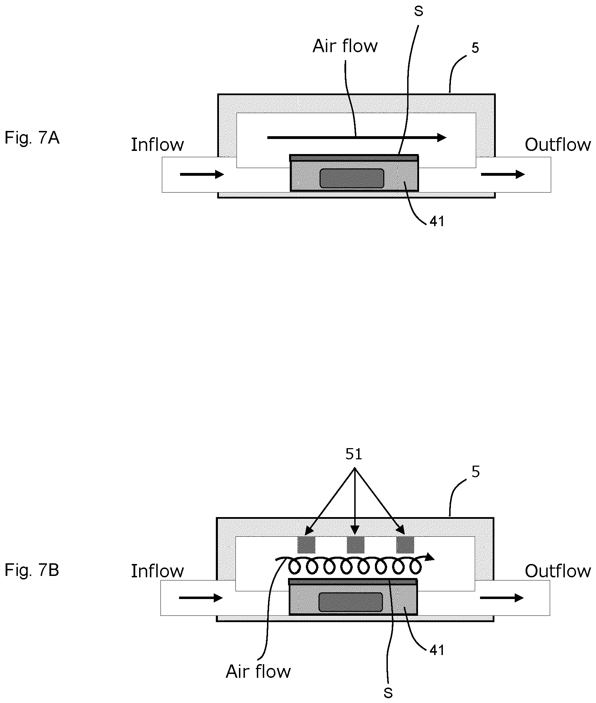

As shown in Fig. 1, Fig. 5, Fig. 6A, and Fig. 6B, the system 100 comprises a covering portion 1 covering the target plant 9, a sensor housing 5, and a sensor 4 configured to alternately measure the carbon dioxide concentration inside and outside the covering portion 1. As shown in Fig. 5, the sensor 4 is provided in the sensor housing 5 in the present embodiment. The sensor 4 comprises a carbon dioxide sensor 41 and a water vapor sensor 42. Here, the carbon dioxide sensor 41 is configured to measure the carbon dioxide concentration inside and outside the covering portion 1. Further, the water vapor sensor 42 is configured to measure the water vapor concentration inside and outside the covering portion 1 in order to calculate a transpiration rate of the plant 9. In addition, as shown in Fig. 7B, an air stirring portion 51 for stirring flow of air flowing into the sensor housing 5 is provided inside the sensor housing 5.

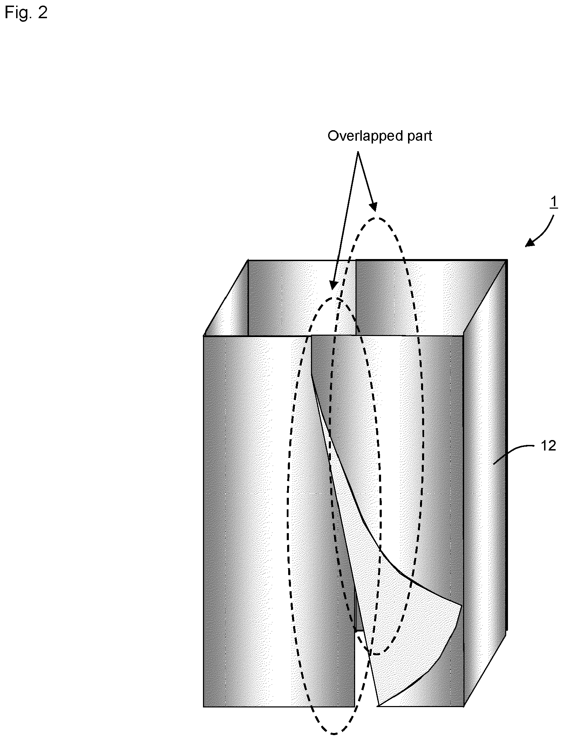

As shown in Fig. 1, Fig. 3, Fig. 4A, and Fig. 4B, the covering portion 1 comprises a canopy 10 and a covering sheet 12. The canopy 10 is arranged above the target plant 9, and the covering sheet 12 is arranged to cover the periphery of the target plant 9. The material of the canopy 10 is not particularly limited, though the material is preferably transparent. For example, the canopy 10 can be formed of a transparent material, such as acrylic resin. Further, as shown in Fig. 2, the covering sheet 12 in the present embodiment is configured to comprise an overlapped part covered by a plurality of sheets. This is mainly for the purpose of preventing air outside the covering portion 1 from flowing into the covering portion 1 through the boundary of a plurality of covering sheets, and also for the purpose of facilitating the care of the plant 9 covered by the covering portion 1. That is, the covering sheet 12 is configured to have the overlapped part covered by the plurality of sheets, so that an operator can flip over the covering sheet 12 in the overlapped part and easily put his or her hand into the covering portion 1, thereby facilitating the care of the plant 9. The overlapped width of the overlapped part is not particularly limited, though the width is preferably 1 cm or more, and more preferably 3 cm or more.

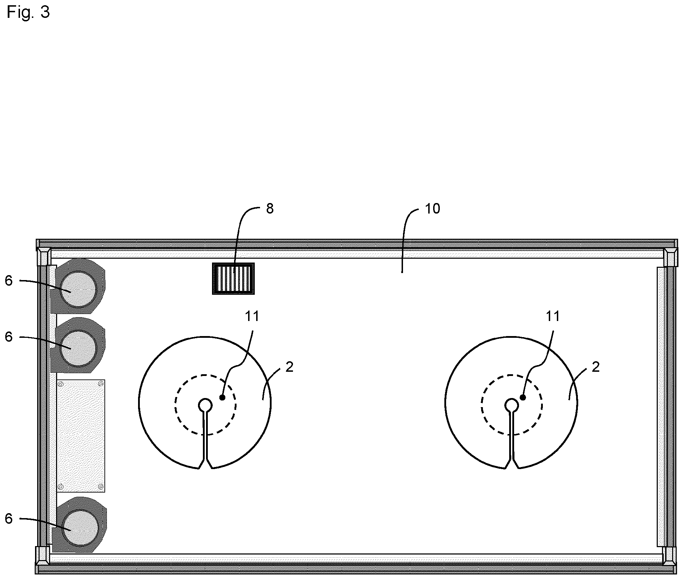

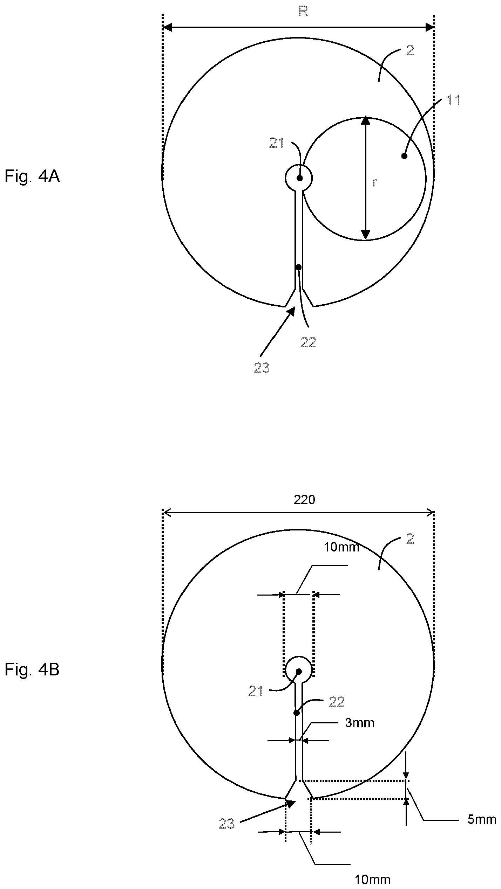

As shown in Fig. 3, Fig. 4A, and Fig. 4B, the canopy 10 is provided with a hole 11, and a lid 2 comprises an insertion portion 21 through which a string 3 can be inserted and a slit 22 extending from an outer edge of the lid 2 to the insertion portion 21. The lid 2 is arranged, in a plan view, such that the insertion portion 21 overlaps the hole 11 and the lid 2 covers the hole 11. As shown in Fig. 1, a wire 33 is arranged above the canopy 10. Further, the string 3 wound around a bobbin 34 attached to the wire 33 is inserted through the hole 11 of the canopy 10 via the insertion portion 21 provided on the lid 2 to hold the plant 9. In the present embodiment, the string 3 outside the bobbin 34 can be wound by the bobbin 34 to be shortened. On the contrary, the string 3 can be pulled out from the bobbin 34, so that the length of the string 3 outside the bobbin 34 can be increased. It is necessary to provide the canopy 10 with the hole 11 in order to suspend the string 3 wound around the bobbin 34 into the covering portion 1. On the other hand, the lid 2 is provided to close the hole 11 in order to measure the carbon dioxide concentration inside the covering portion 1.

As shown in Fig. 4A, the slit 22 in the present embodiment comprises a wide portion 23. The wide portion 23 is configured such that the width of the slit 22 increases toward the outer edge of the lid 2. Such a configuration facilitates the insertion of the string 3 from the wide portion 23 and the insertion of the string 3 through the insertion portion 21 by passing through the slit 22.

As shown in Fig. 4A, the diameter r of the hole 11 and the diameter R of the lid 2 are preferably configured to satisfy the relationship of $2 < R/r$. With such a configuration, the hole 11 can be provided on the canopy 10, and the lid 2 can be arranged to cover the hole 11, so that the string 3 can be inserted through the insertion portion 21, thereby preventing the string 3 from coming off the hole 11. In other words, the hole 11 is always kept closed even when the lid 2 is moved. The value of R/r is, preferably, 2.1 to 3.0, and more preferably, 2.4 to 2.6. Specifically, the value may be, for example, 2.1, 2.2, 2.3, 2.4, 2.5, 2.6, 2.7, 2.8, 2.9, 3.0, and the value may be within a range between any two of the values exemplified herein.

The shape of the lid 2 is not particularly limited as long as the lid 2 can be arranged, in a plan view, such that the insertion portion 21 overlaps the hold 11 and the lid 2 covers the hole 11. For example, as shown in Fig. 4B, the diameter R of the lid 2 can be set to be 220 mm, the diameter of the insertion portion 21 can be set to be 10 mm, the width of the slit 22 can be set to be 3 mm, the width of the wide portion 23 at the outer edge of the lid 2 can be set to be 10 mm, and the length of the wide portion 23 can be set to be 5 mm.

Further, As shown in Fig. 1, a plurality of length adjustment cords 31 attached to the wire 33 is attached to corners of the canopy 10. Each length adjustment cord 31 is provided with a length adjustment portion 32. The length of the length adjustment cord 31 can be adjusted by adjusting the length adjustment portion 32, so that the covering portion 1 can appropriately cover the plant 9 according to the height of the plan 9.

As shown in Fig. 1 and Fig. 3, the canopy 10 is provided with a ventilation fan 6. The ventilation fan 6 penetrates the canopy 10 and is configured to discharge air inside the covering portion 1 to the outside of the covering portion 1. Further, a root holding portion 7 including roots of the plant 9 is provided below the covering sheet 12. The root holding portion 7 comprises rock wool 71 and a slab 72. In this regard, the configuration of the root holding portion 7 is not particularly limited as long as the growth of the plant 9 can be maintained, and the root holding portion 7 may be configured as soil, reservoir or the like. Further, the canopy 10 is provided with a pyranometer 8.

## 2. Sensor 4

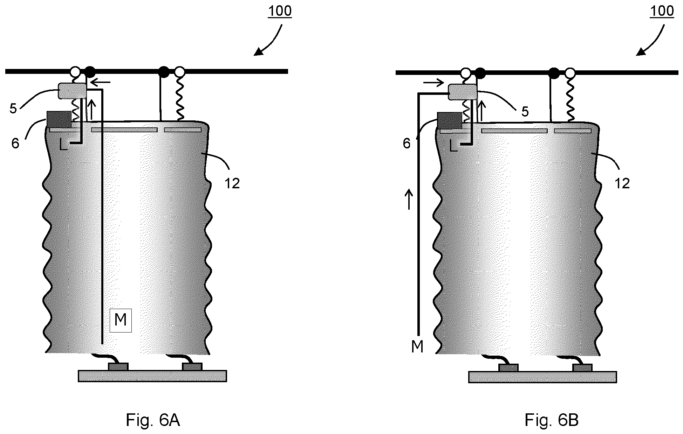

Next, the sensor 4 is described with reference to Fig. 5, Fig. 6A and Fig. 6B. As shown in Fig. 5, the sensor 4 is provided in the sensor housing 5 in the present embodiment. The sensor 4 comprises the carbon dioxide sensor 41 and the water vapor sensor 42. Further, an air pump 43 and a three-way solenoid valve 44 for introducing air into the sensor housing 5 are provided. The three-way solenoid valve 44 is configured to switch the air introduced into the sensor housing 5 between air L or air M, and the air pump 43 is configured to introduce the air L or the air M to a side of the sensor 4. Here, the air M represents air flowing into the covering portion 1 (air outside the covering portion 1). Further, the air L represents air discharged to the outside of the covering portion 1.

In the present embodiment, the carbon dioxide sensor 41 is configured to measure the difference in carbon dioxide concentration between an inflow side of air into the covering portion 1 and an outflow side of air from the covering portion 1. In other words, the carbon dioxide sensor 41 is configured to measure the difference in carbon dioxide concentration between the air flowing into the covering portion 1 and the air discharged to the outside of the covering portion 1. That is, the carbon dioxide sensor 41 is a sensor configured to detect the difference in carbon dioxide concentration. In this regard, the configuration of the carbon dioxide sensor 41 is not limited to this, and a sensor directly detecting the carbon dioxide concentration may be used.

## 3. Air Stirring Portion 51

Next, the air stirring portion 51 is described with reference to Fig. 7A and Fig. 7B. As shown in Fig. 7B, in the present embodiment, the air stirring portion 51 for stirring flow of air flowing in the sensor housing 5 is provided inside the sensor housing 5. The shape of the air stirring portion 51 is not particularly limited, and the air stirring portion 51 configured as a plurality of protrusions is adopted in an example of Fig. 7B. Further, the air stirring portion 51 in the present embodiment is provided inside the sensor housing 5 to face a surface (contact surface S) where the sensor 4 (carbon dioxide sensor 41) is in contact with air. With such configuration, the flow of air is disturbed, and the contact between the air and the sensor 4 (carbon dioxide sensor 41) can be promoted. Consequently, the air stirring portion 51 contributes to improving the response speed of the sensor 4 (carbon dioxide sensor 41) due to change in carbon dioxide concentration when the air introduced into the sensor housing 5 is switched from the air flowing into the covering portion 1 to the air discharged from the covering portion 1 (or from the air discharged from the covering portion 1 to the air flowing into the covering portion 1).

On the other hand, as shown in Fig. 7A, when the air stirring portion 51 is not provided, the flow of air is not stirred, and the contact between the air and the sensor 4 (carbon dioxide sensor 41) is not promoted.

## 4. Necessity of Air Stirring Portion 51

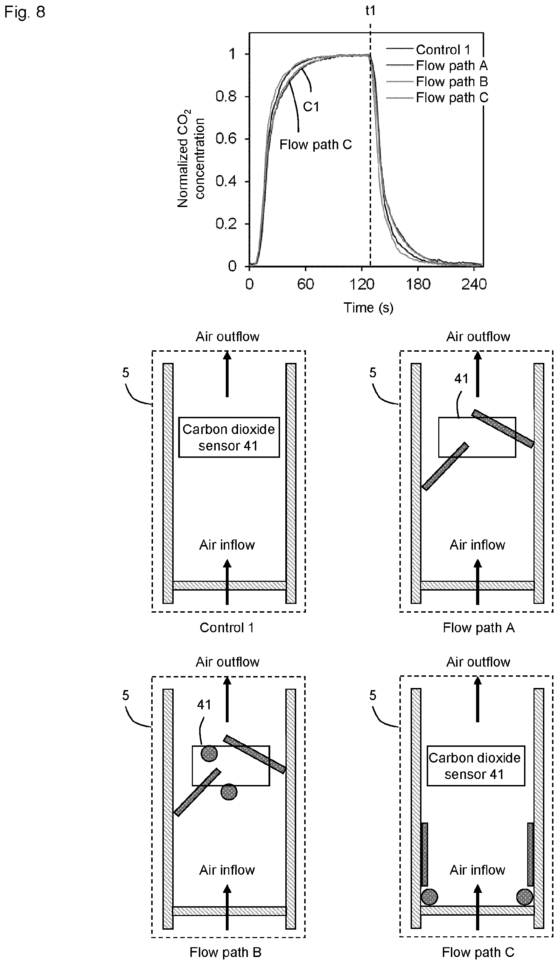

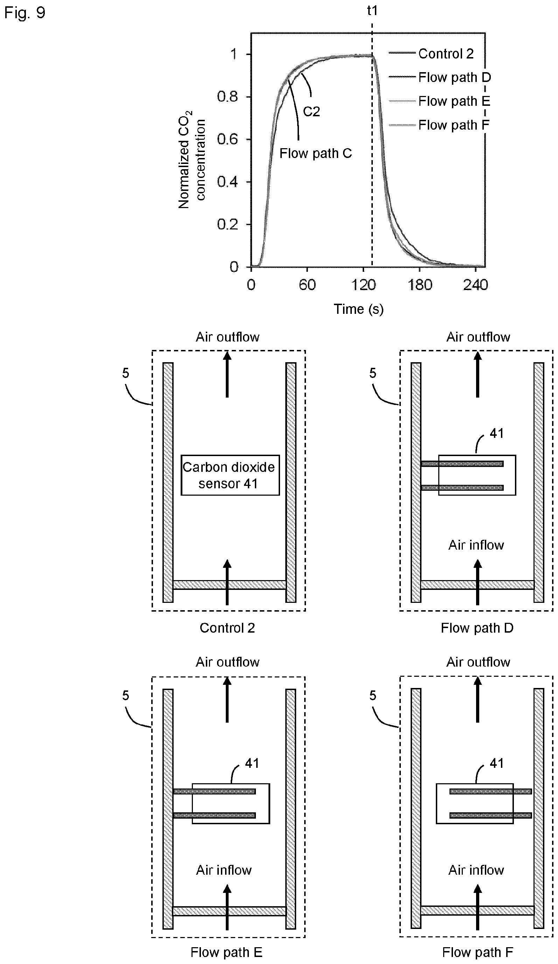

Next, the necessity of the air stirring portion 51 is described with reference to Fig. 8 and Fig. 9. The graph shown in Fig. 8 shows results of experiments conducted on the response speed of the sensor 4 (carbon dioxide sensor 41) for a reference (Control 1) and three comparison targets (flow paths A to C). Specifically, the reference (Control 1) having no air stirring portion 51 on the surface (contact surface S) where the sensor 4 (carbon dioxide sensor 41) is in contact with air, the flow path A and the flow path B provided with the air stirring portion 51 on the surface (contact surface S) where the sensor 4 (carbon dioxide sensor 41) is in contact with air, and the flow path C provided with the air stirring portion 51 in a position apart from the sensor 4 (carbon dioxide sensor 41) were prepared. Fig. 8 shows the normalized value of the carbon dioxide concentration output from the sensor 4 (carbon dioxide sensor 41) when the carbon dioxide concentration of the air introduced into the sensor housing 5 was changed from 380 ppm to 420 ppm. In the example of Fig. 8, the carbon dioxide concentration of the air introduced into the sensor housing 5 was changed from 380 ppm to 420 ppm at time 0, and the carbon dioxide concentration of the air introduced into the sensor housing 5 was changed from 420 ppm to 380 ppm at time t1.

As is clear from the graph shown in Fig. 8, in the case of the reference (Control 1) and the flow path C (C1 and the flow path C in the graph), the response time (from 0 to 90%) until the normalized carbon dioxide concentration reached 0.9 was 46 seconds. On the other hand, in the case of the flow path A and the flow path B, the response time (from 0 to 90%) was 37 seconds. Further, the response time (from 0 to 90%) when the carbon dioxide concentration of the inflow air was changed from 420 ppm to 380 ppm was also the same. The results show that the responsiveness of the sensor 4 (carbon dioxide sensor 41) can be improved by providing the air stirring portion 51 on the surface (the contact surface S) where the sensor 4 (carbon dioxide sensor 41) is in contact with air.

Further, the graph in Fig. 9 shows results of experiments conducted on the response speed of the sensor 4 (carbon dioxide sensor 41) for a reference (Control 2) and three comparison targets (flow paths D to F). Specifically, the reference (Control 2) and the flow path D to the flow path F (corresponding to the air stirring portion 51) provided near the sensor 4 (carbon dioxide sensor 41) were prepared, and Fig. 9 shows the normalized value of the carbon dioxide concentration output from the sensor 4 (carbon dioxide sensor 41) when the carbon dioxide concentration of the air introduced into the sensor housing 5 was changed from 380 ppm to 420 ppm. In the example of Fig. 9, the carbon dioxide concentration of the air introduced into the sensor housing 5 was changed from 380 ppm to 420 ppm at time 0, and the carbon dioxide concentration of the air introduced into the sensor housing 5 was changed from 420 ppm to 380 ppm at time t1.

As is clear from the graph shown in Fig. 9, in the case of the reference (Control 2) (C2 in the graph), the response time (from 0 to 100%) until the normalized carbon dioxide concentration reached 1 was 70 seconds. On the other hand, in the case of the flow path D to the flow path F, the response time (from 0 to 100%) was 60 seconds. Further, in the case of the reference (Control 2) (C2 in the graph), the response time (from 100 to 0%) until the normalized carbon dioxide concentration reached 0 after changing the carbon dioxide concentration of the air introduced into the sensor housing 5 from 420 ppm to 380 ppm was 120 seconds, while the response time (from 100 to 0%) of the flow path D to the flow path F was 80 seconds. This result shows that the responsiveness of the sensor 4 (carbon dioxide sensor 41) is improved by providing the air stirring portion 51 near the sensor 4 (carbon dioxide sensor 41).

As described above, the results in Fig. 8 and Fig. 9 show that the response speed of the sensor 4 (carbon dioxide sensor 41) is improved by providing the air stirring portion 51 near the sensor 4 (carbon dioxide sensor 41).

4. Calculation of Photosynthesis Rate

Next, a method of calculating the photosynthesis rate of the plant 9 using the carbon dioxide concentration measured by the sensor 4 (carbon dioxide sensor 41) is described. A photosynthesis rate A can be calculated, for example, by the following equation.

$$<\text{Equation}> \qquad A = F\,(C_{in} - C_{out})$$

A: photosynthesis rate
F: ventilation rate by the ventilation fan 6 (mol min$^{-1}$)
$C_{in}$: carbon dioxide concentration of the air flowing into the covering portion 1 ($\mu$mol mol$^{-1}$)
$C_{out}$: carbon dioxide concentration of the air discharged from the covering portion 1 ($\mu$mol mol$^{-1}$)

In this way, the photosynthesis rate of the plant 9 can be calculated from the carbon dioxide concentration measured by the sensor 4 (carbon dioxide sensor 41).

5. Others

The system 100 of the present invention can also be implemented in the following aspects.

- The photosynthesis rate of the plant 9 is calculated in real time, and the transition of the photosynthesis is visualized in real time by means of an information processing device.
- When the sunlight is weak and the difference in the carbon dioxide concentration between the air flowing into the covering portion 1 and the air discharged from the covering portion 1 becomes small, a predetermined number (for example, two) of the ventilation fans 6 among a plurality of (for example, three) ventilation fans 6 is stopped.
- Instead of providing a plurality of ventilation fans 6, an ventilation fan whose output can be adjusted is used.

**Reference Signs List**

1       : covering portion

2       : lid

3       : string

4       : sensor

5       : sensor housing

6 : ventilation fan

7 : root holding portion

8 : pyranometer

9 : plant

10 : canopy

11 : hole

12 : covering sheet

21 : insertion portion

22 : slit

23 : wide portion

31 : adjustment cord

32 : adjustment portion

33 : wire

34 : bobbin

41 : carbon dioxide sensor

42 : water vapor sensor

43 : air pump

44 : three-way solenoid valve

51 : air stirring portion

71 : rock wool

72 : slab

100 : system

S : contact surface

**Claims**

1. A photosynthesis rate measurement system of a plant, comprising:

   a covering portion covering a target plant,
   a sensor housing, and
   a sensor in the sensor housing alternately measuring carbon dioxide concentrations inside and outside the covering portion,
   wherein an air stirring portion to enhance the mixing of the air in the sensor housing is equipped inside the sensor housing.

2. The system of Claim 1,
   wherein the sensor measures the difference in carbon dioxide concentrations between inflow air and outflow air of the covering portion, and
   the air stirring portion is provided to improve the response speed of the sensor due to changes in the carbon dioxide concentrations in the air on an inflow side or an outflow side of the air into the covering portion.

3. The system of Claim 1 or Claim 2,
   wherein the air stirring portion is provided to face a surface where the sensor and the air are in contact with each other inside the sensor housing.

4. The system of any one of Claim 1 to Claim 3,
   wherein the covering portion comprises a canopy and a covering sheet,
   the canopy is arranged above the target plant,
   the covering sheet is arranged to cover a side of the target plant, and
   the covering sheet is configured to comprise an overlapped part covered by a plurality of sheets.

5. The system of Claim 4, further comprising a lid and a string,
   wherein the canopy is provided with holes,
   the lid comprises an insertion portion through which the string can be inserted and a slit extending from an outer edge of the lid to the insertion portion,
   the lid is arranged such that the insertion portion overlaps the hole and arranged to cover the hole in a plan view, and
   the string is inserted through the hole via the insertion portion.

6. The system of Claim 5,
   wherein the slit is configured such that slit's width increases toward the edge of the lid.

7. The system of Claim 5 or Claim 6,
   wherein a diameter r of the hole and a diameter R of the lid satisfy a relationship of 2 < R/r.

8. The system of any one of Claim 4 to Claim 7,
   wherein the canopy is provided with a ventilation fan.

Fig. 1

Fig. 2

Overlapped part

1

12

Fig. 3

Fig. 4A

Fig. 4B

Fig. 5

Fig. 6A

Fig. 6B

Fig. 7A

S

Air flow

5

Inflow

Outflow

41

Fig. 7B

51

5

Inflow

Outflow

Air flow

41

S

Fig. 8

Air outflow

Carbon dioxide
sensor 41

Air inflow

Control 1

Air outflow

41

Air inflow

Flow path A

Air outflow

41

Air inflow

Flow path B

Air outflow

Carbon dioxide
sensor 41

Air inflow

Flow path C

Fig. 9

Control 2

Flow path D

Flow path E

Flow path F

**INTERNATIONAL SEARCH REPORT**

| International application No. |
| --- |
| PCT/JP2019/012260 |

**A. CLASSIFICATION OF SUBJECT MATTER**
Int. Cl. A01G7/00(2006.01)i, G01N21/31(2006.01)n

According to International Patent Classification (IPC) or to both national classification and IPC

**B. FIELDS SEARCHED**

Minimum documentation searched (classification system followed by classification symbols)
Int. Cl. A01G7/00, G01N21/00-21/01, G01N21/17-21/61, G01N27/00-27/24

Documentation searched other than minimum documentation to the extent that such documents are included in the fields searched
Published examined utility model applications of Japan      1922-1996
Published unexamined utility model applications of Japan    1971-2019
Registered utility model specifications of Japan            1996-2019
Published registered utility model applications of Japan    1994-2019

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y | Microfilm of the specification and drawings | 1-4, 8 |
| A | annexed to the request of Japanese Utility Model | 5-7 |
| | Application No. 053246/1980 (Laid-open No. 157956/1981) (KOITO INDUSTRIES, LTD.) 25 November 1981, description, page 2, line 16 to page 5, line 20, fig. 2 (Family: none) | |
| Y | JP 7-113775 A (TOYOTA MOTOR CORP.) 02 May 1995, | 1-4, 8 |
| A | paragraphs [0028]-[0031], fig. 9 & US 5531225 A1, column 7, line 56 to column 8, line 10, fig. 9A | 5-7 |
| Y | JP 11-125034 A (KAWASHIMA, Katsuko) 11 May 1999, | 4, 8 |
| A | paragraphs [0012]-[0026], fig. 1 (Family: none) | 5-7 |

☒ Further documents are listed in the continuation of Box C.        ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 04.06.2019 | 18.06.2019 |

| Name and mailing address of the ISA/ Japan Patent Office 3-4-3, Kasumigaseki, Chiyoda-ku, Tokyo 100-8915, Japan | Authorized officer |
| --- | --- |
| | Telephone No. |

Form PCT/ISA/210 (second sheet) (January 2015)

**INTERNATIONAL SEARCH REPORT**

| International application No. |
| --- |
| PCT/JP2019/012260 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y<br>A | JP 2014-226064 A (PIONEER CORP.) 08 December 2014, paragraphs [0068]-[0071], fig. 15 (Family: none) | 8<br>5-7 |
| A | JP 2007-274908 A (JAPAN INTERNATIONAL RESEARCH CENTER FOR AGRICULTURAL SERVICES) 25 October 2007, entire text, all drawings (Family: none) | 1-8 |
| A | US 2014/0026474 A1 (KULAS, Charles J.) 30 January 2014, entire text, all drawings (Family: none) | 1-8 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017044531 A